# EUROPEAN PATENT SPECIFICATION

(11) **EP 3 684 315 B1**
(45) Date of publication and mention of the grant of the patent: **26.03.2025**
(21) Application number: 18783281.1
(22) Date of filing: 20.09.2018
(51) Int. Cl.: A61F 13/511

(54) **ABSORBENT ARTICLE**
ABSORBIERENDER ARTIKEL
ARTICLE ABSORBANT

(30) Priority: 21.09.2017 US 201762561382 P
(43) Date of publication of application: 29.07.2020
(60) Divisional application: 25157761.5
(73) Proprietor: The Procter & Gamble Company, Cincinnati, OH 45202 (US)
(72) Inventor: MCCORMICK, Sarah, Ann, Cincinnati Ohio 45202 (US); KANYA, Kevin, Ronald, Cincinnati Ohio 45202 (US); SAEVECKE, Dirk, 65824 Schwalbach am Taunus (DE); GIOVANNI, Sara, Lyn, Cincinnati Ohio 45202 (US)
(74) Representative: P&G Patent Germany
(86) International application number: PCT/US2018/051897
(87) International publication number: WO 2019/060515

(56) References cited:
- WO-A1-2017/124092
- WO-A1-96/32913
- US-A1- 2007 219 521
- US-A1- 2011 319 849
- US-A1- 2015 374 561

## Description

### FIELD

The present disclosure is generally directed to absorbent articles designed and configured to leverage a greater amount of bio-based materials and/or to minimize the inclusion of unwanted materials towards providing a more pure end product to consumers desiring the same.

### BACKGROUND

The vast majority of commercially available absorbent articles, such as diapers, contain a significant amount of petrochemicals. For example, most mass-produced diapers include fibrous outer layers that contain at least some petroleum-based fibers and a liquid barrier layer made from a petroleum-based film. This segment of absorbent articles also typically contain lotions on the wearer-facing surface, and include fragrances or perfumes for positive scent experiences. There are however a few small manufacturers that are beginning to offer absorbent articles that are touted to be "eco-friendly," with benefits for the environment and/or the wearer of the absorbent article. Some eco-friendly diapers are chlorine-free, lotion-free, and fragrance free, so as to be more "natural" of a product. Unsurprisingly, the eco-friendly diapers focus on benefits to the wearer; for example, they can contain a wearer-facing surface that is free of lotions and include natural fibers or other materials. However, caregivers of minors or non-ambulatory adults also have significant contact with the absorbent articles from the non-wearer facing surface as they hold the minors and/or change the absorbent articles. Thus, there is need for absorbent articles that contain natural or bio-based materials and/or that are devoid of unwanted materials in close proximity of both of its outer-facing surfaces and not just the wearer-facing surface.

As noted above, some commercially-available eco-friendly diapers are fragrance free as perfume raw materials and fragrance compositions can be skin sensitizers to some individuals. Without any fragrance added to the diapers however, the remaining fibrous and film-based components can have an inherent odor that, while not unsafe for wearers of the diapers, can be off- putting to caregivers. Thus, there is need for absorbent articles that contain bio-based materials and/or that are devoid of masking fragrances that also do not have an undesirable odor.

US 2007/219521 A1 and US 2011/319849 A1 disclose an absorbent article comprising a synthetic polymer derived from a renewable resource and methods of producing the article.

US 2015/374561 A1 discloses a heterogeneous mass containing foam.

US 2014/005621 A1 discloses a bio-based absorbent article including a topsheet, a backsheet, and an absorbent core.

US 2017/056253 A1 discloses an absorbent insert for use with a wearable absorbent article.

WO 2017/124092 A1 discloses a composite structure including at least one natural fiber web layer and at least one nonwoven web layer.

The invention provides an absorbent article according to claim 1. Optional features of the invention are set out in the dependent claims.

### BRIEF DESCRIPTION OF THE DRAWINGS

The above-mentioned and other features and advantages of the present disclosure, and the manner of attaining them, will become more apparent and the disclosure itself will be better understood by reference to the following description of example forms of the disclosure taken in conjunction with the accompanying drawings, wherein:
Fig. 1 is a plan view of an example absorbent article in the form of a taped diaper, garment-facing surface facing the viewer, in a flat laid-out state;
Fig. 2 is a plan view of the example absorbent article of Fig. 1, wearer-facing surface facing the viewer, in a flat laid-out state;
Fig. 3 is a front perspective view of the absorbent article of Figs. 1 and 2 in a fastened position;
Fig. 4 is a front perspective view of an absorbent article in the form of a pant;
Fig. 5 is a rear perspective view of the absorbent article of Fig. 4;
Fig. 6 is a plan view of the absorbent article of Fig. 4, laid flat, with a garment-facing surface facing the viewer;
Fig. 7 is a cross-sectional view of the absorbent article taken about line 7-7 of Fig. 6;
Fig. 8 is a cross-sectional view of the absorbent article taken about line 8-8 of Fig. 6;
Fig. 9 is a plan view of an example absorbent core or an absorbent article;
Fig. 10 is a cross-sectional view, taken about line 10-10, of the absorbent core of Fig. 9;
Fig. 11 is a cross-sectional view, taken about line 11-11, of the absorbent core of Fig. 10;
Fig. 12 is a plan view of an example absorbent article of the present disclosure that is a sanitary napkin;
Fig. 13 is an example cross-sectional view taken within a front waist region of an absorbent article;
Fig. 14 is an example cross-sectional view taken within a crotch region of an absorbent article;
Fig. 15 is an example cross-sectional view taken within a back waist region of an absorbent article; and
Fig. 16 is a side view of a package containing a plurality of absorbent articles.

### DETAILED DESCRIPTION

Various non-limiting forms of the present disclosure will now be described to provide an overall understanding of the principles of the structure, function, manufacture, and use of the absorbent articles disclosed herein. One or more examples of these non-limiting forms are illustrated in the accompanying drawings. Those of ordinary skill in the art will understand that the absorbent articles described herein and illustrated in the accompanying drawings are non-limiting example forms and that the scope of the various non-limiting forms of the present disclosure are defined solely by the claims. The features illustrated or described in connection with one non-limiting form may be combined with the features of other non-limiting forms. Such modifications and variations are intended to be included within the scope of the present disclosure.

### Definitions

"Absorbent article" means devices that absorb and/or contain liquid. Wearable absorbent articles are absorbent articles placed against or in close proximity to the body of the wearer to absorb and contain various exudates discharged from the body. Non-limiting examples of wearable absorbent articles include diapers, pant-like or pull-on diapers, training pants, sanitary napkins, pantiliners, incontinence devices (liners, pads, and briefs), and the like.

"Animal-based fibers" includes wool, hair, and secretions, such as silk.

"Bio-based content" refers to the amount of carbon from a renewable resource in a material as a percent of the mass of the total organic carbon in the material, as determined by ASTM D6866-10, method B. In order to apply the methodology of ASTM D6866-10 to determine the bio-based content of any absorbent article or component thereof, a sample can be ground into particulates less than about 20 mesh using known grinding methods (e.g., WILEY mill), and a representative sample of suitable mass taken from the randomly mixed particles. Alternatively, if the sample is merely a layer of material, then the layer itself can be analyzed without the need for a pre-grinding step. Note that any carbon from inorganic sources such as calcium carbonate is not included in determining the bio-based content of the material. See additional information below for measuring bio-sourced content in polymers. Components of the absorbent articles described in this specification can at least partially be comprised of bio-sourced content as described in the following U.S. published patent applications nos.: US 2007/0219521, US 2011/0139658, US 2011/0139657, US 2011/0152812, US 2011/0139662, and US 2011/0139659.

Absorbent articles of the present disclosure may be "devoid of" or "free of'' particular undesirable materials, ingredients, or characteristics in some forms. "Devoid of," "free of," and the like, as those terms are used herein, can mean the absorbent article does not have more than trace amounts of background levels of the material, ingredient, or characteristic following these qualifiers; the amount of the material or ingredient does not cause harm or irritation that consumers typically associate with the material or ingredient; or the material or ingredient was not added to the absorbent article intentionally. In some instances, "devoid of" and "free of" can mean there is no measurable amount of the material or ingredient. For example, the absorbent article in some forms contain no measurable amounts of chlorine-that is, the article is characterized as being totally chlorine free.

"Naturally-derived" materials includes materials that are partially chemically altered without petroleum components and that have been minimally processed such that they not be altered to such an extent that they are substantially less biodegradable or more toxic.

"Natural fibers" refers to elongated substances produced by plants and animals and includes animal-based fibers and plant-based fibers, as those categories are described herein. Natural fibers, as that term is used herein, include fibers harvested without any post-harvest treatment step as well as those having a post-treatment step, such as, for example, washing, scouring, bleaching.

"Plant-based fibers", as that term is used herein, includes both harvested fibers and synthetic fibers that comprise bio-based content. Harvested plant-based fibers include cellulosic matter, such as wood pulp; seed hairs, such as cotton; stem (or bast) fibers, such as flax and hemp; leaf fibers, such as sisal; and husk fibers, such as coconut.

"Renewable resource" refers to a natural resource that can be replenished within a 100 year time frame. The resource may be replenished naturally, or via agricultural techniques. Renewable resources include plants, animals, fish, bacteria, fungi, and forestry products. They may be naturally occurring hybrids, or genetically engineered organisms.

### Validation of Polymers Derived from Renewable Resources

A suitable validation technique is through ¹⁴C analysis. A small amount of the carbon dioxide in the atmosphere is radioactive. This ¹⁴C carbon dioxide is created when nitrogen is struck by an ultra-violet light produced neutron, causing the nitrogen to lose a proton and form carbon of molecular weight 14 which is immediately oxidized to carbon dioxide. This radioactive isotope represents a small but measurable fraction of atmospheric carbon. Atmospheric carbon dioxide is cycled by green plants to make organic molecules during photosynthesis. The cycle is completed when the green plants or other forms of life metabolize the organic molecules, thereby producing carbon dioxide which is released back to the atmosphere. Virtually all forms of life on Earth depend on this green plant production of organic molecules to grow and reproduce. Therefore, the ¹⁴C that exists in the atmosphere becomes part of all life forms, and their biological products. In contrast, fossil fuel based carbon does not have the signature radiocarbon ratio of atmospheric carbon dioxide.

Assessment of the renewably based carbon in a material can be performed through standard test methods. Using radiocarbon and isotope ratio mass spectrometry analysis, the bio-based content of materials can be determined. ASTM International, formally known as the American Society for Testing and Materials, has established a standard method for assessing the bio-based content of materials. The ASTM method is designated ASTM D6866-10.

The application of ASTM D6866-10 to derive a "bio-based content" is built on the same concepts as radiocarbon dating, but without use of the age equations. The analysis is performed by deriving a ratio of the amount of organic radiocarbon (¹⁴C) in an unknown sample to that of a modern reference standard. The ratio is reported as a percentage with the units "pMC" (percent modern carbon).

The modern reference standard used in radiocarbon dating is a NIST (National Institute of Standards and Technology) standard with a known radiocarbon content equivalent approximately to the year AD 1950. AD 1950 was chosen since it represented a time prior to thermo-nuclear weapons testing which introduced large amounts of excess radiocarbon into the atmosphere with each explosion (termed "bomb carbon"). The AD 1950 reference represents 100 pMC.

"Bomb carbon" in the atmosphere reached almost twice normal levels in 1963 at the peak of testing and prior to the treaty halting the testing. Its distribution within the atmosphere has been approximated since its appearance, showing values that are greater than 100 pMC for plants and animals living since AD 1950. It's gradually decreased over time with today's value being near 107.5 pMC. This means that a fresh biomass material such as corn could give a radiocarbon signature near 107.5 pMC.

Combining fossil carbon with present day carbon into a material will result in a dilution of the present day pMC content. By presuming 107.5 pMC represents present day biomass materials and 0 pMC represents petroleum derivatives, the measured pMC value for that material will reflect the proportions of the two component types. A material derived 100% from present day soybeans would give a radiocarbon signature near 107.5 pMC. If that material was diluted with 50% petroleum derivatives, for example, it would give a radiocarbon signature near 54 pMC (assuming the petroleum derivatives have the same percentage of carbon as the soybeans).

A bio-based content result is derived by assigning 100% equal to 107.5 pMC and 0% equal to 0 pMC. In this regard, a sample measuring 99 pMC will give an equivalent bio-based content value of 92%.

Assessment of the materials described herein can be done in accordance with ASTM D6866. The mean values quoted in this report encompasses an absolute range of 6% (plus and minus 3% on either side of the bio-based content value) to account for variations in end-component radiocarbon signatures. It is presumed that all materials are present day or fossil in origin and that the bio-based content result is the amount of bio-based component "present" in the material.

### General Description of an Absorbent Article

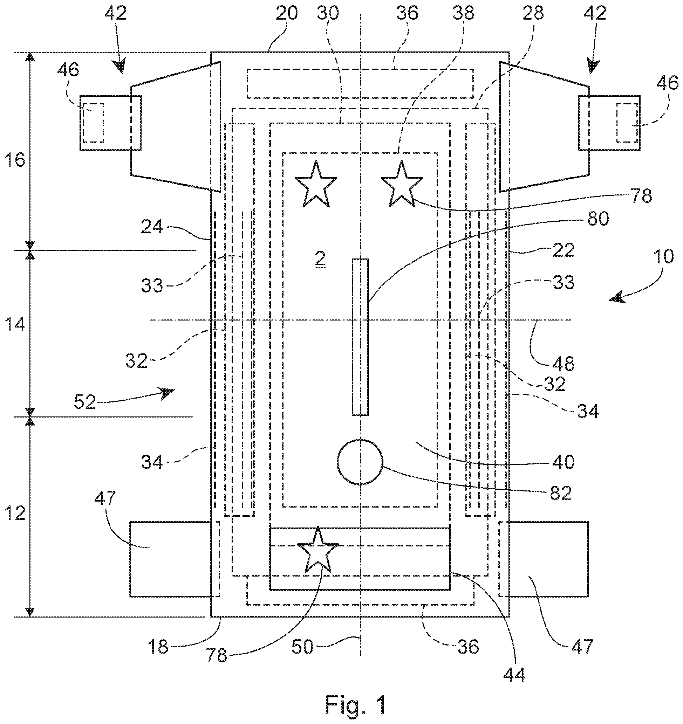

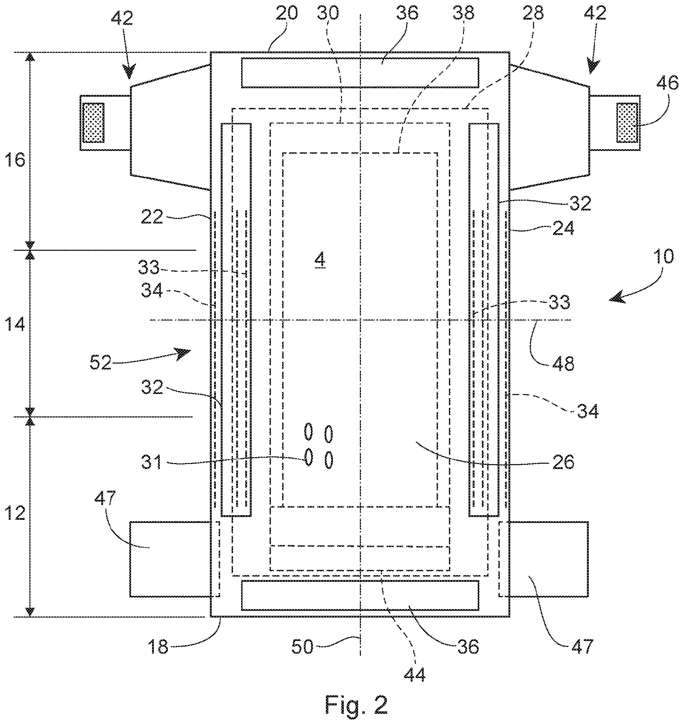

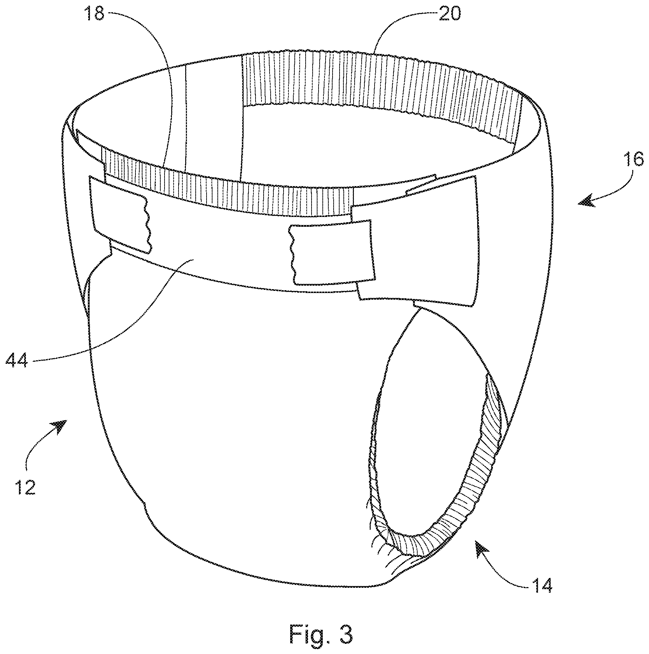

An example absorbent article 10 according to the present disclosure, shown in the form of a taped diaper, is represented in Figs. 1-3. Fig. 1 is a plan view of the example absorbent article 10, garment-facing surface 2 facing the viewer in a flat, laid-out state (i.e., no elastic contraction). Fig. 2 is a plan view of the example absorbent article 10 of Fig. 1, wearer-facing surface 4 facing the viewer in a flat, laid-out state. Fig. 3 is a front perspective view of the absorbent article 10 of Figs. 1 and 2 in a fastened configuration. The absorbent article 10 of Figs. 1-3 is shown for illustration purposes only as the present disclosure may be used for making a wide variety of diapers, including adult incontinence products, pants, or other absorbent articles, such as sanitary napkins and absorbent pads, for example.

The absorbent article 10 may comprise a front waist region 12, a crotch region 14, and a back waist region 16. The crotch region 14 may extend intermediate the front waist region 12 and the back waist region 16. The front wait region 12, the crotch region 14, and the back waist region 16 may each be 1/3 of the length of the absorbent article 10. The absorbent article 10 may comprise a front end edge 18, a back end edge 20 opposite to the front end edge 18, and longitudinally extending, transversely opposed side edges 22 and 24 defined by the chassis 52.

The absorbent article 10 may comprise a liquid permeable topsheet 26, a liquid impermeable backsheet 28, and an absorbent core 30 positioned at least partially intermediate the topsheet 26 and the backsheet 28. The absorbent article 10 may also comprise one or more pairs of barrier leg cuffs 32 with or without elastics 33, one or more pairs of leg elastics 34, one or more elastic waistbands 36, and/or one or more acquisition materials 38. The acquisition material or materials 38 may be positioned intermediate the topsheet 26 and the absorbent core 30. An outer cover material 40, such as a nonwoven material, may cover a garment-facing side of the backsheet 28. The absorbent article 10 may comprise back ears 42 in the back waist region 16. The back ears 42 may comprise fasteners 46 and may extend from the back waist region 16 of the absorbent article 10 and attach (using the fasteners 46) to the landing zone area or landing zone material 44 on a garment-facing portion of the front waist region 12 of the absorbent article 10. The absorbent article 10 may also have front ears 47 in the front waist region 12. The absorbent article 10 may have a central lateral (or transverse) axis 48 and a central longitudinal axis 50. The central lateral axis 48 extends perpendicular to the central longitudinal axis 50.

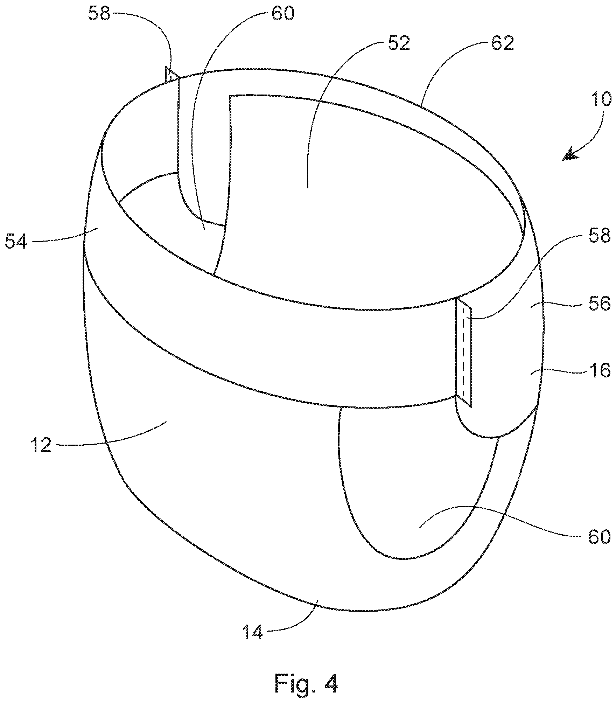

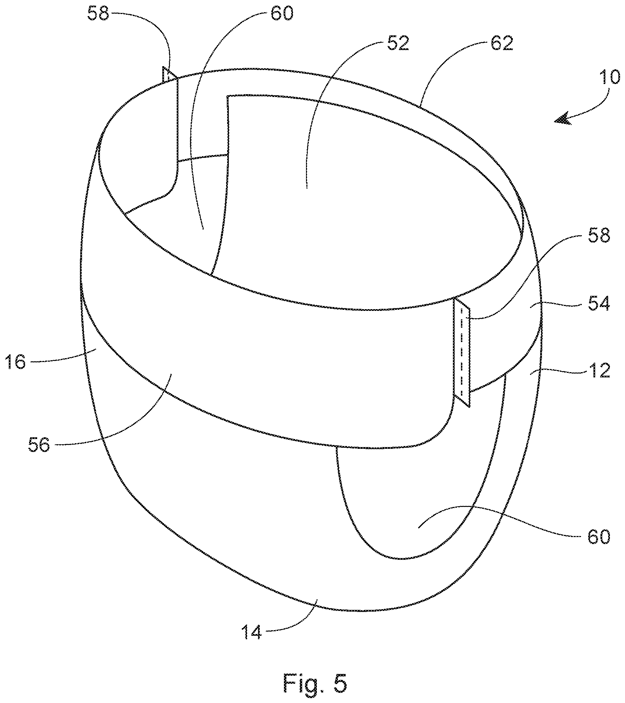

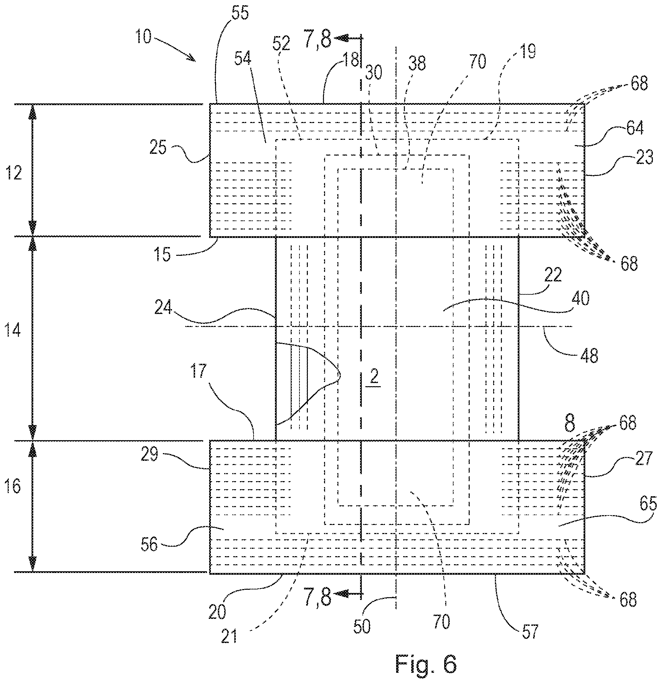

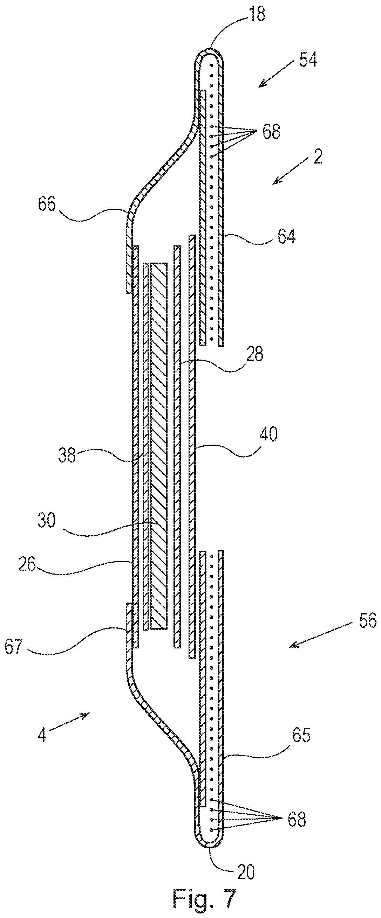

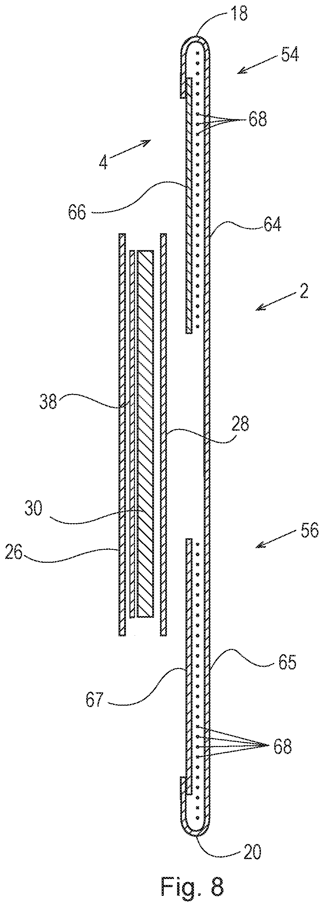

In other instances, the absorbent article may be in the form of a pant having permanent or refastenable side seams. Suitable refastenable seams are disclosed in U.S. patent application publication no. US 2014/0005020 and U.S. Patent no. 9,421,137. Referring to Figs. 4-8, an example absorbent article 10 in the form of a pant is illustrated. Fig. 4 is a front perspective view of the absorbent article 10. Fig. 5 is a rear perspective view of the absorbent article 10. Fig. 6 is a plan view of the absorbent article 10, laid flat, with the garment-facing surface facing the viewer. Elements of Fig. 4-8 having the same reference number as described above with respect to Figs. 1-3 may be the same element (e.g., absorbent core 30). Fig. 7 is an example cross-sectional view of the absorbent article taken about line 7-7 of Fig. 6. Fig. 8 is an example cross-sectional view of the absorbent article taken about line 8-8 of Fig. 6. Figs. 7 and 8 illustrate example forms of front and back belts 54, 56. The absorbent article 10 may have a front waist region 12, a crotch region 14, and a back waist region 16. Each of the regions 12, 14, and 16 may be 1/3 of the length of the absorbent article 10. The absorbent article 10 may have a chassis 52 (sometimes referred to as a central chassis or central panel) comprising a topsheet 26, a backsheet 28, and an absorbent core 30 disposed at least partially intermediate the topsheet 26 and the backsheet 28, and an optional acquisition material 38, similar to that as described above with respect to Figs. 1-3. The absorbent article 10 may comprise a front belt 54 in the front waist region 12 and a back belt 56 in the back waist region 16. The chassis 52 may be joined to a wearer-facing surface 4 of the front and back belts 54, 56 or to a garment-facing surface 2 of the belts 54, 56. Side edges 23 and 25 of the front belt 54 may be joined to side edges 27 and 29, respectively, of the back belt 56 to form two side seams 58. The side seams 58 may be any suitable seams known to those of skill in the art, such as butt seams or overlap seams, for example. When the side seams 58 are permanently formed or refastenably closed, the absorbent article 10 in the form of a pant has two leg openings 60 and a waist opening circumference 62. The side seams 58 may be permanently joined using adhesives or bonds, for example, or may be refastenably closed using hook and loop fasteners, for example.

### Belts

Referring to Figs. 7 and 8, the front and back belts 54 and 56 may comprise front and back inner belt layers 66 and 67 and front and back outer belt layers 64 and 65 having an elastomeric material (e.g., strands 68 or a film (which may be apertured)) disposed at least partially therebetween. The elastic elements 68 or the film may be relaxed (including being cut) to reduce elastic strain over the absorbent core 30 or, may alternatively, run continuously across the absorbent core 30. The elastics elements 68 may have uniform or variable spacing therebetween in any portion of the belts. The elastic elements 68 may also be pre-strained the same amount or different amounts. The front and/or back belts 54 and 56 may have one or more elastic element free zones 70 where the chassis 52 overlaps the belts 54, 56. In other instances, at least some of the elastic elements 68 may extend continuously across the chassis 52.

The front and back inner belt layers 66, 67 and the front and back outer belt layers 64, 65 may be joined using adhesives, heat bonds, pressure bonds or thermoplastic bonds. Various suitable belt layer configurations can be found in U.S. patent application publication no. US 2013/0211363.

Front and back belt end edges 55 and 57 may extend longitudinally beyond the front and back chassis end edges 19 and 21 (as shown in Fig. 6) or they may be co-terminus. The front and back belt side edges 23, 25, 27, and 29 may extend laterally beyond the chassis side edges 22 and 24. The front and back belts 54 and 56 may be continuous (i.e., having at least one layer that is continuous) from belt side edge to belt side edge (e.g., the transverse distances from 23 to 25 and from 27 to 29). Alternatively, the front and back belts 54 and 56 may be discontinuous from belt side edge to belt side edge (e.g., the transverse distances from 23 to 25 and 27 to 29), such that they are discrete.

As disclosed in U.S. Patent no. 7,901,393, the longitudinal length (along the central longitudinal axis 50) of the back belt 56 may be greater than the longitudinal length of the front belt 54, and this may be particularly useful for increased buttocks coverage when the back belt 56 has a greater longitudinal length versus the front belt 54 adjacent to or immediately adjacent to the side seams 58.

The front outer belt layer 64 and the back outer belt layer 65 may be separated from each other, such that the layers are discrete or, alternatively, these layers may be continuous, such that a layer runs continuously from the front belt end edge 55 to the back belt end edge 57. This may also be true for the front and back inner belt layers 66 and 67 - that is, they may also be longitudinally discrete or continuous. Further, the front and back outer belt layers 64 and 65 may be longitudinally continuous while the front and back inner belt layers 66 and 67 are longitudinally discrete, such that a gap is formed between them - a gap between the front and back inner and outer belt layers 64, 65, 66, and 67 is shown in Fig. 7 and a gap between the front and back inner belt layers 66 and 67 is shown in Fig. 8.

The front and back belts 54 and 56 may include slits, holes, and/or perforations providing increased breathability, softness, and a garment-like texture. Underwear-like appearance can be enhanced by substantially aligning the waist and leg edges at the side seams 58 (see Figs. 4 and 5).

The front and back belts 54 and 56 may comprise graphics (see e.g., 78 of Fig. 1). The graphics may extend substantially around the entire circumference of the absorbent article 10 and may be disposed across side seams 58 and/or across proximal front and back belt seams 15 and 17; or, alternatively, adjacent to the seams 58, 15, and 17 in the manner described in U.S. Pat. No. 9,498, 389 to create a more underwear-like article. The graphics may also be discontinuous.

Alternatively, instead of attaching belts 54 and 56 to the chassis 52 to form a pant, discrete side panels may be attached to side edges of the chassis 22 and 24. Suitable forms of pants comprising discrete side panels are disclosed in U.S. Patent nos. 6,645,190; 8,747,379; 8,372,052; 8,361,048; 6,761,711; 6,817,994; 8,007,485; 7,862,550; 6,969,377; 7,497,851; 6,849,067; 6,893,426; 6,953,452; 6,840,928; 8,579,876; 7,682,349; 7,156,833; and 7,201,744.

### Topsheet

The topsheet 26 is the outermost layer of the absorbent article 10 that is in contact with the wearer's skin. The topsheet 26 may be joined to portions of the backsheet 28, the absorbent core 30, the barrier leg cuffs 32, and/or any other layers as is known to those of ordinary skill in the art. The topsheet 26 may be compliant, soft-feeling, and non-irritating to the wearer's skin. Further, at least a portion of, or all of, the topsheet may be liquid permeable, permitting liquid bodily exudates to readily penetrate through its thickness. A suitable topsheet may be manufactured from a wide range of materials, such as porous foams, reticulated foams, apertured plastic films, woven materials, nonwoven materials, woven or nonwoven materials of natural fibers (e.g., wood or cotton fibers), synthetic fibers or filaments (e.g., polyester or polypropylene or bicomponent PE/PP fibers or mixtures thereof), or a combination of natural and synthetic fibers.

The topsheet may be a nonwoven material having one or multiple formed layers comprising plant-based fibers other than wood pulp. The nonwoven can be made through well-known techniques; for example, the nonwoven can be a spunbond, or be a carded and air-through or calendar bonded nonwoven. The plant-based fibers can also be spun fibers made at least in part from bio-based materials. For example, the plant-based fibers can be spun starch fibers or bio-based polyolefin spun fibers. And the plant-based fibers can be single component fibers or multicomponent fibers wherein less than all of the components are plant-based fibers. One example is a bi-component fiber comprising a polyester core component and a bio-based polyethylene sheath component.

The topsheet may have one or more layers. The topsheet may be apertured (Fig. 2, element 31), may have any suitable three-dimensional features, and/or may have a plurality of embossments (e.g., a bond pattern). The topsheet may be apertured by overbonding a material and then rupturing the overbonds through ring rolling, such as disclosed in U.S. Patent no. 5,628,097, to Benson et al., issued on May 13, 1997 and disclosed in U.S. patent application publication no. US 2016/0136014 to Arora et al.

The topsheet may be a dual layer, spunbond nonwoven web. Each of the layers has a basis weight from about 10 gsm to about 25 gsm or 35 gsm. One layer may have similar or different hydrophilicity/hydrophobicity profiles compared to the other layer. **In** another form the topsheet is an air through carded nonwoven web comprising a blend (e.g., 50-50 weight percent) of polyester fibers and bio-based polyethylene fibers. The basis weight of such a nonwoven is typically about 20 gsm, 25 gsm, 35 gsm, or 40 gsm.

Many commercially-available absorbent articles comprise a skin care composition on at least a portion of the topsheet; and these compositions tend to contain petrolatum or other petroleum-based materials as their main component. The topsheet may be devoid of a lotion or skin care composition. However, a skin care composition or lotion can optionally be added to the topsheet. And if one is, it is preferred, but not required, to employ a composition based upon natural or naturally-derived materials such as fats, oils or waxes, for example. The optional lotions can comprise vegetable oils, algae oils, bacterial derived oils, and animal fats), combinations of theses, and the like. Representative examples of vegetable oils include argan oil, canola oil, rapeseed oil, coconut oil, corn oil, cottonseed oil, olive oil, palm oil, peanut oil, safflower oil, sesame oil, soy-bean oil, sunflower oil, high oleoyl soy-bean oil, high oleoyl sunflower oil, linseed oil, palm kernel oil, tung oil, castor oil, high oloeyl sunflower oil, high oleoyl soybean oil, high erucic rape oils, Jatropha oil, combinations of theses, and the like. Representative examples of animal fats include lard, tallow, chicken fat, yellow grease, fish oil, combinations of these, and the like. A representative example of a synthesized oil includes tall oil, which is a byproduct of wood pulp manufacture. Additional suitable lotion compositions derived from renewable resources are disclosed in U.S. patent application publication no. US 2013/0144239.

The topsheet may be hydrophilic or hydrophobic or may have hydrophilic and/or hydrophobic portions or layers. If the topsheet is hydrophobic, typically apertures will be present so that bodily exudates may pass through the topsheet.

### Backsheet

The backsheet 28 is generally that portion of the absorbent article 10 positioned proximate to the garment-facing surface of the absorbent core 30. The backsheet 28 may be joined to portions of the topsheet 26, the outer cover material 40, the absorbent core 30, and/or any other layers of the absorbent article by any attachment methods known to those of skill in the art. The backsheet 28 prevents, or at least inhibits, the bodily exudates absorbed and contained in the absorbent core 10 from soiling articles such as bedsheets, undergarments, and/or clothing. The backsheet is typically liquid impermeable, or at least substantially liquid impermeable. The backsheet may, for example, be or comprise a thin plastic film, such as a thermoplastic film having a thickness of about 0.012 mm to about 0.051 mm. Other suitable backsheet materials may include breathable materials which permit vapors to escape from the absorbent article, while still preventing, or at least inhibiting, bodily exudates from passing through the backsheet. Backsheet films can optionally comprise bio-based materials, such as, for example, bio-based polyethylene.

### Outer Cover Material

The outer cover material (sometimes referred to as a backsheet nonwoven) 40 may comprise one or more nonwoven materials joined to the backsheet 28 and that covers the backsheet 28. The outer cover material is typically the outermost layer facing outward-i.e., towards garments or undergarments when present and away from the wearer-s skin. A caregiver interacts significantly with the outer cover material when holding/comforting the wearer and/or changing the absorbent articles. The outer cover material 40 forms at least a portion of the garment-facing surface 2 of the absorbent article 10 and effectively "covers" the backsheet 28 so that film is not present on the garment-facing surface 2. The outer cover material 40 may comprise a bond pattern, apertures, and/or three-dimensional features. The outer cover material may comprise a carded nonwoven or a multi-layered nonwoven comprising carded layers and one or more spunbond layers.

The nonwoven can comprise a combination of plant-based fibers and synthetic fibers that are not plant-based. For example, the nonwoven can comprise both polypropylene fibers and cotton fibers; see, for example, U.S. patent application publication no. US 2017/0203542. The cotton content can range from about 3%, 5%, 10%, or 15% to about 50%, by weight of the nonwoven. When synthetic fibers such as polypropylene are employed, it is preferred that the polypropylene be non-phthalate catalyst polypropylene fibers.

The outer cover material may comprise a hydroentangled, dual layer nonwoven web, wherein one layer comprises a polypropylene spunbond web having a basis weight in the range of from about 5 to 15 gsm, and the other layer comprises a carded nonwoven web comprising cotton fibers and a basis weight in the range of from about 10 to about 25 gsm. The outer cover material may comprise an air through carded nonwoven web comprising a blend of polypropylene fibers and cotton fibers. The cotton fibers can be included in these nonwoven webs in an amount of about 5%, 10%, or 15% to about 20%, 30%, or 50%, by weight of the overall nonwoven web.

### Absorbent Core

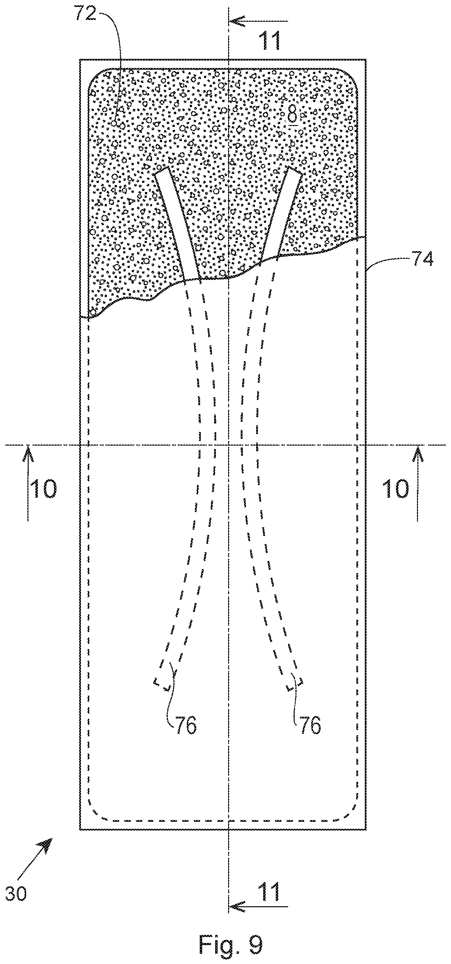

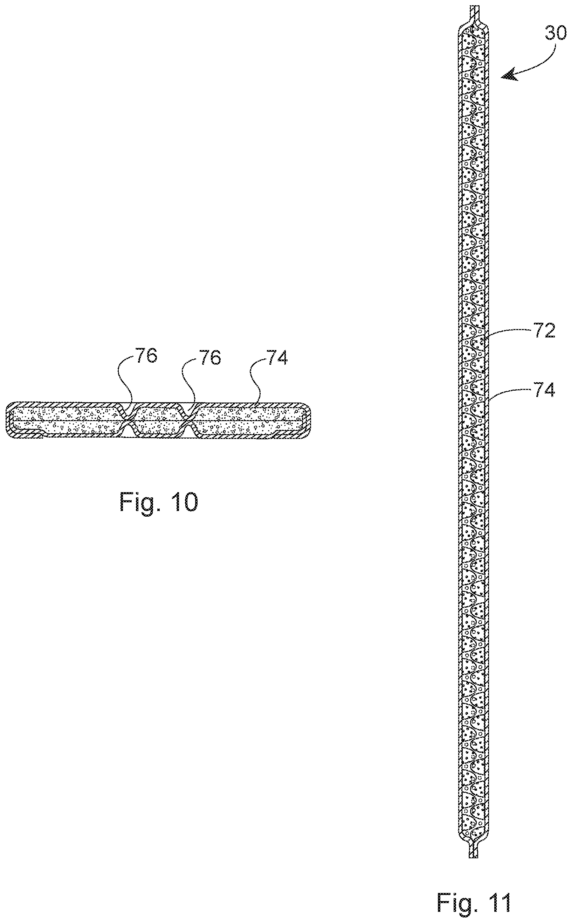

As used herein, the term "absorbent core" 30 refers to the component of the absorbent article 10 having the most absorbent capacity and that comprises an absorbent material. Referring to Figs. 9-11, in some instances, absorbent material 72 may be positioned within a core bag or a core wrap 74. The absorbent material may be profiled or not profiled, depending on the specific absorbent article. The absorbent core 30 may comprise, consist essentially of, or consist of, a core wrap, absorbent material 72, and glue enclosed within the core wrap. The core bag or core wrap may be constructed with one or more nonwoven webs. These nonwoven webs can comprise non-phthalate catalyst polymers. The webs can also comprise bio-based materials, including plant-based fibers.

The absorbent material may comprise superabsorbent polymers (also known as absorbent gelling materials of "agm"), a mixture of superabsorbent polymers and air felt, only air felt, and/or a high internal phase emulsion foam. In some instances, the absorbent material may comprise at least 80%, at least 85%, at least 90%, at least 95%, at least 99%, or up to 100% superabsorbent polymers, by weight of the absorbent material. In such instances, the absorbent material may free of air felt, or at least mostly free of air felt. The absorbent core periphery, which may be the periphery of the core wrap, may define any suitable shape, such as rectangular "T," "Y," "hour-glass," or "dog-bone" shaped, for example. An absorbent core periphery having a generally "dog bone" or "hour-glass" shape may taper along its width towards the crotch region 14 of the absorbent article 10.

Referring to Figs. 9-11, the absorbent core 30 may have areas having little or no absorbent material 72, where a wearer-facing surface of the core bag 74 may be joined to a garment-facing surface of the core bag 74. These areas having little or no absorbent material may be referred to as "channels" 76. These channels can embody any suitable shapes and any suitable number of channels may be provided. In other instances, the absorbent core may be embossed to create the impression of channels. The absorbent core in Figs. 9-11 is merely an example absorbent core. Many other absorbent cores with or without channels are also within the scope of the present disclosure.

In some particular examples, the superabsorbent polymers of the core absorbent material may comprise a bio-based acrylic acid. Bio-based acrylic acid and methods of production are further described in U.S. patent application publication no. US 2007/0219521 and U.S. Patent nos. 8,703,450 and 9,630,901. The superabsorbent polymers of the present disclosure may have a bio-based content of from about 5% to about 100%, from about 10% to about 100%, from about 25% to about 100%, from about 40% to about 100%, from about 50% to about 100%, from about 75% to about 100%, or from about 90% to about 100%.

### Barrier Leg Cuffs/Leg Elastics

Referring to Figs. 1 and 2, for example, the absorbent article 10 may comprise one or more pairs of barrier leg cuffs 32 and one or more pairs of leg elastics 34. The barrier leg cuffs 32 may be positioned laterally inboard of leg elastics 34. Each barrier leg cuff 32 may be formed by a piece of material which is bonded to the absorbent article 10 so it can extend upwards from a wearer-facing surface 4 of the absorbent article 10 and provide improved containment of body exudates approximately at the junction of the torso and legs of the wearer. Useful materials include, but are not limited to, spunbond-meltblown-spunbond (SMS) nonwoven webs. Such webs can comprise non-phthalate catalyst polypropylene fibers, and/or plant-based fibers.

The barrier leg cuffs 32 are delimited by a proximal edge joined directly or indirectly to the topsheet and/or the backsheet and a free terminal edge, which is intended to contact and form a seal with the wearer's skin. The barrier leg cuffs 32 may extend at least partially between the front end edge 18 and the back end edge 20 of the absorbent article 10 on opposite sides of the central longitudinal axis 50 and may be at least present in the crotch region 14.

The barrier leg cuffs 32 may each comprise one or more elastics 33 (e.g., elastic strands or strips) near or at the free terminal edge. These elastics 33 cause the barrier leg cuffs 32 to help form a seal around the legs and torso of a wearer. The leg elastics 34 extend at least partially between the front end edge 18 and the back end edge 20. The leg elastics 34 essentially cause portions of the absorbent article 10 proximate to the chassis side edges 22, 24 to help form a seal around the legs of the wearer. The leg elastics 34 may extend at least within the crotch region 14. The leg elastics preferably are latex-free.

### Elastic Waistband

Referring to Figs. 1 and 2, the absorbent article 10 may comprise one or more elastic waistbands 36. The elastic waistbands 36 may be positioned on the garment-facing surface 2 or the wearer-facing surface 4. As an example, a first elastic waistband 36 may be present in the front waist region 12 near the front belt end edge 18 and a second elastic waistband 36 may be present in the back waist region 16 near the back end edge 20. The elastic waistbands 36 may aid in sealing the absorbent article 10 around a waist of a wearer and at least inhibiting bodily exudates from escaping the absorbent article 10 through the waist opening circumference. In some instances, an elastic waistband may fully surround the waist opening circumference of an absorbent article.

### Acquisition/Distribution Materials

Referring to Figs. 1, 2, 7, and 8, one or more acquisition and/or distribution materials 38 may be present at least partially intermediate the topsheet 26 and the absorbent core 30. The acquisition/distribution materials 38 are typically hydrophilic materials that provide significant wicking of bodily exudates. These materials may dewater the topsheet 26 and quickly move bodily exudates into the absorbent core 30. The acquisition/distribution materials 38 may comprise one or more nonwoven materials, foams, cellulosic materials, cross-linked cellulosic materials, air laid cellulosic nonwoven materials, spunlace materials, or combinations thereof, for example. The cellulosic materials and cross-linked cellulosic materials may be bleached, but preferably not via chlorine-bleaching so as to be total chlorine free. Hydrogen peroxide is one exemplary bleaching material that is useful in making acquisition/distribution materials that are totally chlorine-free.

In some instances, portions of the acquisition/distribution materials 38 may extend through portions of the topsheet 26, portions of the topsheet 26 may extend through portions of the acquisition materials 38, and/or the topsheet 26 may be nested with the acquisition/distribution materials 38. Typically, an acquisition/distribution material 38 may have a width and length that are smaller than the width and length of the topsheet 26. The acquisition/distribution material may be a secondary topsheet in the feminine pad context. The acquisition/distribution material may have one or more channels as described above with reference to the absorbent core 30 (including the embossed version). The channels in the acquisition/distribution material may align or not align with channels in the absorbent core 30. In an example, a first acquisition/distribution material may comprise a nonwoven material and as second acquisition/distribution material may comprise a cross-linked cellulosic material.

### Landing Zone

Referring to Figs. 1 and 2, the absorbent article 10 may have a landing zone area 44 that is formed in a portion of the garment-facing surface 2 of the outer cover material 40. The landing zone area 44 may be in the back waist region 16 if the absorbent article 10 fastens from front to back or may be in the front waist region 12 if the absorbent article 10 fastens back to front. In some instances, the landing zone 44 may be or may comprise one or more discrete nonwoven materials that are attached to a portion of the outer cover material 40 in the front waist region 12 or the back waist region 16 depending upon whether the absorbent article fastens in the front or the back. In essence, the landing zone 44 is configured to receive the fasteners 46 and may comprise, for example, a plurality of loops configured to be engaged with, a plurality of hooks on the fasteners 46, or *vice versa.* Landing zone nonwovens and fastener hooks may contain polymers containing bio-based content.

### Wetness Indicator/Graphics

Referring to Fig. 1, the absorbent articles 10 of the present disclosure may comprise graphics 78 and/or wetness indicators 80 that are visible from the garment-facing surface 2. The graphics 78 may be printed on the landing zone 40, the backsheet 28, and/or at other locations. The wetness indicators 80 are typically applied to the absorbent core facing side of the backsheet 28, so that they can be contacted by bodily exudates within the absorbent core 30. In some instances, the wetness indicators 80 may form portions of the graphics 78. For example, a wetness indicator may appear or disappear and create/remove a character within some graphics. In other instances, the wetness indicators 80 may coordinate (e.g., same design, same pattern, same color) or not coordinate with the graphics 78.

The wetness indicator may comprise a color changing composition based upon a pH change when contacted with a chemical compound typically contained in urine. The color changing composition can be devoid of poly aromatic hydrocarbons.

### Front and Back Ears

Referring to Figs. 1 and 2, as referenced above, the absorbent article 10 may have front and/or back ears 47, 42 in a taped diaper context. Only one set of ears may be required in most taped diapers. The single set of ears may comprise fasteners 46 configured to engage the landing zone or landing zone area 44. If two sets of ears are provided, in most instances, only one set of the ears may have fasteners 46, with the other set being free of fasteners. The ears, or portions thereof, may be elastic or may have elastic panels. In an example, an elastic film or elastic strands may be positioned intermediate a first nonwoven material and a second nonwoven material. The nonwoven materials may comprise non-phthalate catalyst polypropylene fibers.

The elastic film may or may not be apertured. The ears may be shaped. The ears may be integral (e.g., extension of the outer cover material 40, the backsheet 28, and/or the topsheet 26) or may be discrete components attached to a chassis 52 of the absorbent article on a wearer-facing surface 4, on the garment-facing surface 2, or intermediate the two surfaces 4, 2.

### Odor Management Materials

Absorbent articles of the present disclosure may employ odor management materials. These materials may manage odors inherit to the raw materials used in the manufacture of the absorbent articles and/or manage odors associated with absorbed material (e.g., urine, feces, menses). The odor management materials may be devoid of perfume raw materials or fragrances as these traditional materials can be irritants to some individuals. The odor management materials can include natural extracts, naturally derived materials, and/or mineral-based materials. Exemplary odor management materials encompassed within these categories includes activated carbon, zeolites, silica, and combinations thereof.

### Adhesives

Adhesives can be employed to affix one component to another component in the article's final assembly. Adhesives can also be employed to immobilize sub-components, such as, for example, particulate matter within an absorbent core component. The adhesives in some instances are devoid of added fluorescence.

### Sensors

Referring again to Fig. 1, the absorbent articles of the present disclosure may comprise a sensor system 82 for monitoring changes within the absorbent article 10. The sensor system 82 may be discrete from or integral with the absorbent article 10. The absorbent article 10 may comprise sensors that can sense various aspects of the absorbent article 10 associated with insults of bodily exudates such as urine and/or BM (e.g., the sensor system 82 may sense variations in temperature, humidity, presence of ammonia or urea, various vapor components of the exudates (urine and feces), changes in moisture vapor transmission through the absorbent articles garment-facing layer, changes in translucence of the garment-facing layer, and/or color changes through the garment-facing layer). Additionally, the sensor system 82 may sense components of urine, such as ammonia or urea and/or byproducts resulting from reactions of these components with the absorbent article 10. The sensor system 82 may sense byproducts that are produced when urine mixes with other components of the absorbent article 10 (e.g., adhesives, agm). The components or byproducts being sensed may be present as vapors that may pass through the garment-facing layer. It may also be desirable to place reactants in the absorbent article that change state (e.g. color, temperature) or create a measurable byproduct when mixed with urine or BM. The sensor system 82 may also sense changes in pH, pressure, odor, the presence of gas, blood, a chemical marker or a biological marker or combinations thereof. The sensor system 82 may have a component on or proximate to the absorbent article that transmits a signal to a receiver more distal from the absorbent article, such as an iPhone, for example. The receiver may output a result to communicate to the caregiver a condition of the absorbent article 10. In other instances, a receiver may not be provided, but instead the condition of the absorbent article 10 may be visually or audibly apparent from the sensor on the absorbent article.

### Packages

The absorbent articles of the present disclosure may be placed into packages. The packages may comprise polymeric bag and an optional carton surrounding at least a portion of the polymeric bag. The polymeric bag can comprise bio-based polyolefin. The optional carton can be made of fiberboard and can contain recycled material or a blend of recycled and virgin material. Each package may comprise a plurality of absorbent articles.

Communication in the form of graphics and/or indicia relating to properties of the absorbent articles may be formed on, printed on, positioned on, and/or placed on outer portions of the packages. For example, the communication may relate to the absence of certain undesirable materials, such as chlorine, perfume, scent, fragrance, lotion, non-phthalate-catalyst polypropylene, adhesives having added florescence, and green number 7 dye. The communication may also relate to features of the contained absorbent articles, such as, that the absorbent article has cotton (via cotton seal icon) or plant-based materials.

The absorbent articles may be packed under compression so as to reduce the size of the packages, while still providing an adequate amount of absorbent articles per package. By packaging the absorbent articles under compression, caregivers can easily handle and store the packages, while also providing distribution savings to manufacturers owing to the size of the packages.

Accordingly, packages of the absorbent articles of the present disclosure may have an In-Bag Stack Height of less than about 110 mm, less than about 105 mm, less than about 100 mm, less than about 95 mm, less than about 90 mm, less than about 85 mm, less than about 80 mm, less than about 78 mm, less than about 76 mm, less than about 74 mm, less than about 72mm, or less than about 70 mm, specifically reciting all 0.1 mm increments within the specified ranges and all ranges formed therein or thereby, according to the In-Bag Stack Height Test described herein. Alternatively, packages of the absorbent articles of the present disclosure may have an In-Bag Stack Height of from about 70 mm to about 110 mm, from about 70 mm to about 105 mm, from about 70 mm to about 100 mm, from about 70 mm to about 95 mm, from about 70 mm to about 90 mm, from about 70 mm to about 85 mm, from about 72 mm to about 80 mm, or from about 74 mm to about 78 mm, specifically reciting all 0.1 mm increments within the specified ranges and all ranges formed therein or thereby, according to the In-Back Stack Height Test described herein.

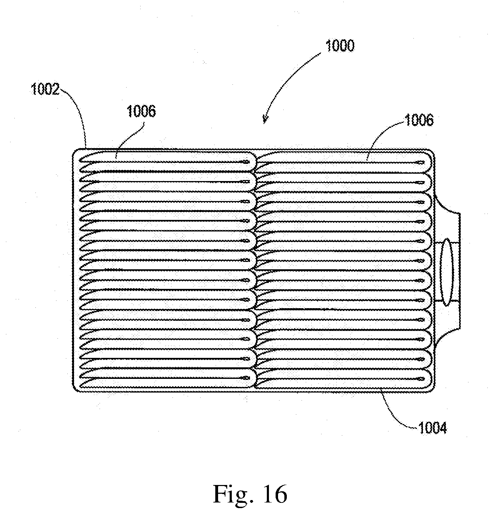

Fig. 16 illustrates an example package 1000 comprising a plurality of absorbent articles 1004. The package 1000 defines an interior space 1002 in which the plurality of absorbent articles 1004 are situated. The plurality of absorbent articles 1004 are arranged in one or more stacks 1006.

### Arrays

"Array" means a display of packages comprising disposable absorbent articles of different article constructions (e.g., different elastomeric materials [compositionally and/or structurally] in the side panels, side flaps and/or belts flaps, different graphic elements, different product structures, fasteners or lack thereof). The packages may have the same brand and/or sub-brand and/or the same trademark registration and/or having been manufactured by or for a common manufacturer and the packages may be available at a common point of sale (e.g. oriented in proximity to each other in a given area of a retail store). An array is marketed as a line-up of products normally having like packaging elements (e.g., packaging material type, film, paper, dominant color, design theme, etc.) that convey to consumers that the different individual packages are part of a larger line-up. Arrays often have the same brand, for example, "Huggies," and same sub-brand, for example, "Pull-Ups." A different product in the array may have the same brand "Huggies" and the sub-brand "Little Movers." The differences between the "Pull-Ups" product of the array and the "Little Movers" product in the array may include product form, application style, different fastening designs or other structural elements intended to address the differences in physiological or psychological development. Furthermore, the packaging is distinctly different in that "Pull-Ups" is packaged in a predominately blue or pink film bag and "Little Movers" is packaged in a predominately red film bag.

Further regarding "Arrays," as another example an array may be formed by different products having different product forms manufactured by the same manufacturer, for example, "Kimberly-Clark", and bearing a common trademark registration for example, one product may have the brand name "Huggies," and sub-brand, for example, "Pull-Ups." A different product in the array may have a brand/sub-brand "Good Nites" and both are registered trademarks of The Kimberly-Clark Corporation and/or are manufactured by Kimberly-Clark. Arrays also often have the same trademarks, including trademarks of the brand, sub-brand, and/or features and/or benefits across the line-up. "On-line Array" means an "Array" distributed by a common on-line source.

### Sanitary Napkin

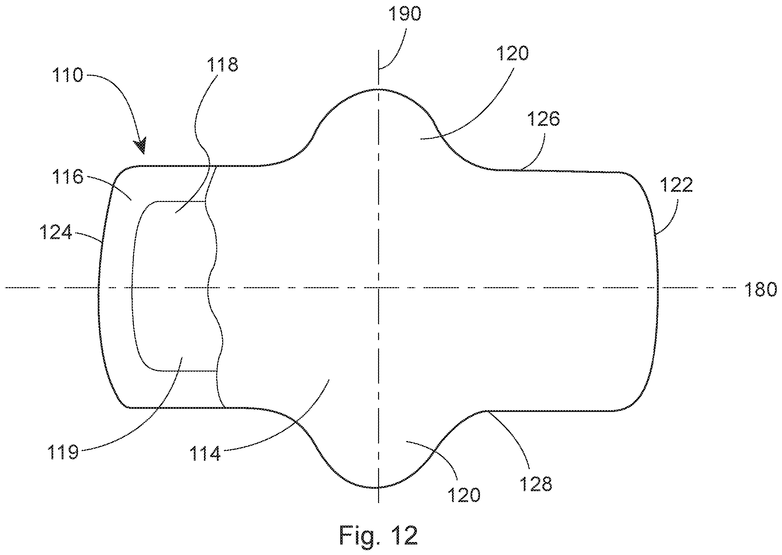

Referring to Fig. 12, an absorbent article of the present disclosure may be a sanitary napkin 110. The sanitary napkin 110 may comprise a liquid permeable topsheet 114, a liquid impermeable, or substantially liquid impermeable, backsheet 116, and an absorbent core 118. The liquid impermeable backsheet 116 may or may not be vapor permeable. The absorbent core 118 may have any or all of the features described herein with respect to the absorbent core 30 and, in some forms, may have a secondary topsheet 119 (STS) instead of the acquisition materials disclosed above. The STS 119 may comprise one or more channels, as described above (including the embossed version). Channels in the STS 119 may be aligned with channels in the absorbent core 118. The sanitary napkin 110 may also comprise wings 120 extending outwardly with respect to a longitudinal axis 180 of the sanitary napkin 110. The sanitary napkin 110 may also comprise a lateral axis 190. The wings 120 may be joined to the topsheet 114, the backsheet 116, and/or the absorbent core 118. The sanitary napkin 110 may also comprise a front edge 122, a back edge 124 longitudinally opposing the front edge 122, a first side edge 126, and a second side edge 128 longitudinally opposing the first side edge 126. The longitudinal axis 180 may extend from a midpoint of the front edge 122 to a midpoint of the back edge 124. The lateral axis 190 may extend from a midpoint of the first side edge 128 to a midpoint of the second side edge 128. The sanitary napkin 110 may also be provided with additional features commonly found in sanitary napkins as is known in the art.

### Examples Cross-sections of Absorbent Articles

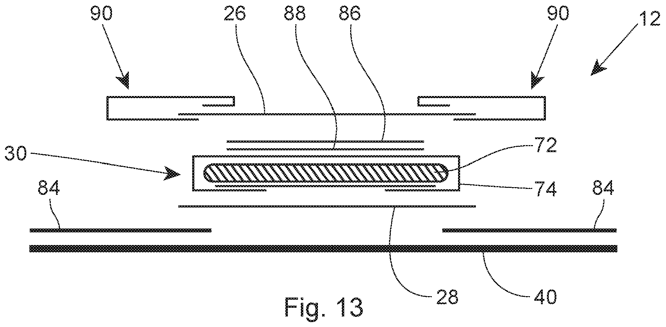

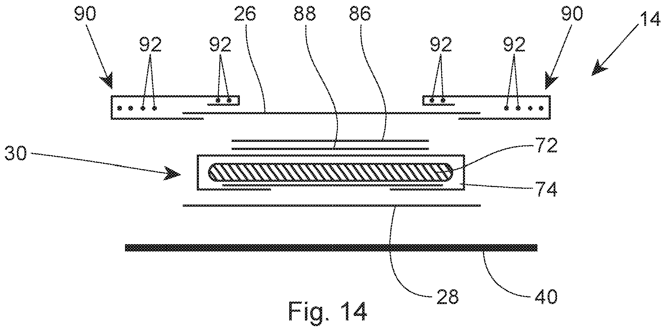

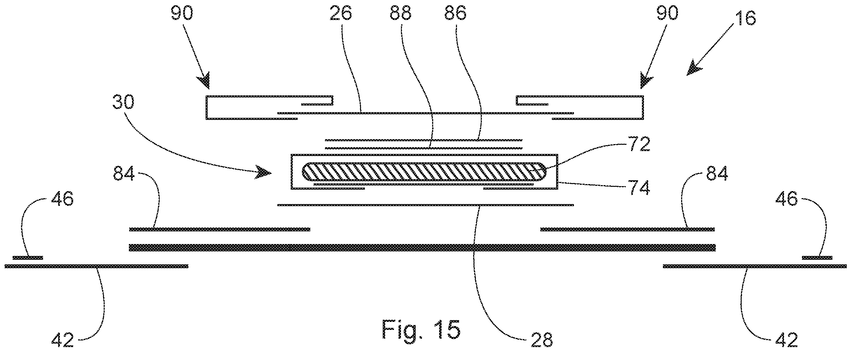

Figs. 13-15 illustrate example cross-sectional views of absorbent articles within the scope of the present disclosure. Fig. 13 is an example cross-sectional view taken within a front waist region 12 of an absorbent article. Fig. 14 is an example cross-sectional view taken within a crotch region 14 of an absorbent article. Fig. 15 is an example cross-sectional view taken within a back waist region 16 of an absorbent article. In Figs. 13-15, an outer cover material is element 40, a liquid permeable topsheet is element 26, opacity patches are elements 84, a liquid impermeable backsheet is element 28, an absorbent core is element 30, with the core bag being element 74, an absorbent material is element 72, and a distribution material is element 86. The distribution material 86 may comprise cross-linked cellulosic material and may be optional. An acquisition material is element 88. A liquid permeable topsheet is element 26. Barrier leg cuffs are elements 90. Elastics in the barrier leg cuffs are elements 92. Back ears are elements 42. Fasteners on the back ears 42 are elements 46. Construction glues and/or bonds between the various layers and/or components have been removed for clarity. Other cross-sectional configurations known to those of skill in the art are also within the scope of the present disclosure.

### Examples

| **Absorbent Article Component** | **Material** | **Material** |
|---|---|---|
| Topsheet | Dual layer spunbond nonwoven web (25 gsm/25 gsm), with each layer comprising bi-component fibers having a | 35 gsm air through carded nonwoven web comprising 50/50 blend, based on weight percent, of polyester fibers and bio-based polyethylene fibers |
| | polypropylene core and bio-based polyethylene sheath | No Lotion |
| | Top layer: hydrophobic Bottom layer: hydrophilic | |
| | No Lotion | |
| Acquisition Layer | 43 gsm carded nonwoven comprising coarse (>4 dtex) PET fibers using a styrenebutadiene binder | 33 gsm carded nonwoven comprising PET fibers (>1 dtex) using a styrenebutadiene binder |
| Distribution Layer | Hydrogen peroxide bleached cellulosic material | Hydrogen peroxide bleached cellulosic material |
| Absorbent Core | Two nonwoven layers and absorbent gelling material disposed therebetween | Two nonwoven layers and absorbent gelling material disposed therebetween |
| Odor Management | 0.15 g of silica mixed with gelling material in the absorbent core | 0.15 g of silica mixed with gelling material in the absorbent core |
| | No perfume/fragrance | No perfume/fragrance |
| Backsheet | Polyolefin film | Polyolefin film |
| Outer Cover Material | 27-30 gsm hybrid nonwoven comprising a spunbond layer and one or more carded layers, the layers being integrated via hydroentangling, and the hybrid nonwoven comprising polypropylene fibers and 15 weight percent cotton fibers | 20-27 gsm air through carded nonwoven comprising polypropylene fibers and 3 to 5 weight percent cotton fibers |
| | | Nonwoven can optionally be apertured |
| | Nonwoven can optionally be apertured | |

The absorbent articles of the present disclosure may be free of, or devoid of, paraben, latex, perfumes, and/or fragrances. Adhesives used in the absorbent articles may be free of, or devoid of, fluorescence. Inks used in the absorbent articles may be free of, or devoid of, green number 7 dye.

The first and second outermost layers of the absorbent articles disclosed herein may be three-dimensional apertured materials, such as those disclosed in U.S. Patent Application Publication No. 2015/0250662, published on September 10, 2015, to Olaf Erik Isele et al. Such a three-dimensional apertured material may form a topsheet and/or an outer cover material of an absorbent article. In an outer cover context, the three-dimensional material may or may not be apertured.

The first and second outermost layers of the absorbent articles disclosed herein may be apertured materials, such as those disclosed in U.S. Patent Application Publication No. 2016/0136014, published on May 19, 2016, to Kelyn Anne Arora et al. Such an apertured material may form a topsheet and/or an outer cover material of an absorbent article.

The first and/or second outmost layers of the absorbent articles disclosed herein may or may not comprise plant-based fibers. The first and/or second outermost layers of the absorbent articles disclosed herein may or may not comprise cotton.

Cotton and/or plant-based fibers may be included in any of the layers of the absorbent articles, such as a core wrap, an acquisition material, a distribution material, ears, and/or other nonwoven components of an absorbent article.

The first and second outermost layers of the absorbent articles disclosed herein may comprise cotton, but may not comprise bio-based content. One of the first and second outermost layers of the absorbent articles disclosed herein may comprise cotton and be free of bio-based content, while the other of the first and second outermost layers may comprise bio-based content and cotton.

### Topsheet Examples:

1. Bicomponent sheath/core fibers; Sheath: Bio Polyethylene 50% by weight of fibers; Core regular polypropylene 50% by weight of fibers; Apertured topsheet
2. Bicomponent sheath/core fibers; Sheath: Bio Polyethylene 50% by weight of fibers; Core regular Polyethylene terphathalate 50% by weight of fibers; Three-dimensional, apertured topsheet
3. Bicomponent sheath/core fibers; Sheath: Bio Polyethylene 50% by weight of fibers; Core regular polypropylene 50% by weight of fibers; 5% cotton by weight; Apertured topsheet
4. Bicomponent sheath/core fibers; Sheath: Bio Polyethylene 50% by weight of fibers; Core Polylactic acid PLA 50% by weight of fibers; Apertured topsheet
5. Bicomponent sheath/core fibers; Sheath: Bio Polyethylene 50% by weight of fibers; Core Polylactic acid 50% by weight of fibers; Three-dimensional, apertured topsheet

### Outer Cover Nonwoven Material Examples:

1. Polyethylene (10%)/Polypropylene (40%)/Polyethylene terphathalate (35%)/cotton (15%) with a total basis weight of 27 gsm.
2. 85% Bio Polylactic acid by weight and 15% cotton by weight

### Test Methods: Basis Weight, Density and Water Retention

Measurements are conducted at 23 °C ± 2 C° and 50% ± 2% relative humidity. All samples are conditioned at this environment for 2 hours prior to testing. Harvest the substrate/component/material of interest from an absorbent article. From the longitudinal and lateral centerline of the sample, accurately cut a specimen of approximately 10 cm² to the nearest 0.01 cm². Measure the mass of the specimen and record as the dry mass to the nearest 0.0001 g. Basis weight is calculated from the measured mass (g) and area (m²) and recorded to the nearest 0.1 g/m² (gsm).

Caliper is measured using a digital caliper such as an Ono Sokki linear gauge sensor GS-503 capable of measuring thickness to ± 0.001 mm. A 25.0 mm ± 0.1 mm diameter circular foot which applies a pressure of 0.69 kPa ± 0.01 kPa is used. The caliper anvil is larger than the foot. The instrument is calibrated per the manufactures specifications. With the foot resting on the anvil, zero the digital caliper. Lift the caliper foot and center the specimen under the foot. Gently lower the foot onto the surface of the specimen at a rate of approximately 2 mm/s. Read and record to the nearest 0.01 mm.

Calculate the volume of the specimen using the measured area (cm²) and caliper (cm), and record to the nearest 0.01 cm³. Density is calculated by dividing the measured mass (g) by the measured volume (cm³). Record to the nearest 0.01 g/cm³.

Take the specimen and submerge it in distilled water for 5.0 ± 0.1 min. Remove the specimen from the water, carefully suspend the specimen vertically from its corner for 10 ± 0.1 min to allow to drain. Measure the mass of the specimen and record as the wet mass to the nearest 0.0001 g. Calculate the Water Retention as the difference between the wet mass and dry mass divided by the dry mass (g). Record to the nearest 0.01 g/g.

In like fashion, repeat the measurements for a total of 10 replicate specimens, and report results as the arithmetic average for Basis Weight (g/m²), Density (g/cm³) and Water Retention (g/g).

### Test Method: The in-bag stack height of a package of articles is determined as follows:

### Equipment

A thickness tester with a flat, rigid horizontal sliding plate is used. The thickness tester is configured so that the horizontal sliding plate moves freely in a vertical direction with the horizontal sliding plate always maintained in a horizontal orientation directly above a flat, rigid horizontal base plate. The thickness tester includes a suitable device for measuring the gap between the horizontal sliding plate and the horizontal base plate to within ± 0.5 mm. The horizontal sliding plate and the horizontal base plate are larger than the surface of the absorbent article package that contacts each plate, i.e. each plate extends past the contact surface of the absorbent article package in all directions. The horizontal sliding plate exerts a downward force of 850 ± 1 gram-force (8.34 N) on the absorbent article package, which may be achieved by placing a suitable weight on the center of the non-package-contacting top surface of the horizontal sliding plate so that the total mass of the sliding plate plus added weight is 850 ± 1grams.

Absorbent article packages are equilibrated at 23 ± 2 °C and 50 ± 5 % relative humidity prior to measurement.

The horizontal sliding plate is raised and an absorbent article package is placed centrally under the horizontal sliding plate in such a way that the absorbent articles within the package are in a horizontal orientation (see Fig. 16). Any handle or other packaging feature on the surfaces of the package that would contact either of the plates is folded flat against the surface of the package so as to minimize their impact on the measurement. The horizontal sliding plate is lowered slowly until it contacts the top surface of the package and then released. The gap between the horizontal plates is measured to within ± 0.5 mm ten seconds after releasing the horizontal sliding plate. Five identical packages (same size packages and same absorbent articles counts) are measured and the arithmetic mean is reported as the package width. The "In-Bag Stack Height" = (package width/absorbent article count per stack) × 10 is calculated and reported to within ± 0.5 mm.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document cited herein, the meaning or definition assigned to that term in this document shall govern.

While particular embodiments of the present disclosure have been illustrated and described, it would be obvious to those skilled in the art that various other changes and modifications can be made without departing from the scope of the claims. It is therefore intended to cover in the appended claims all such changes and modifications that are within the scope of the claims.

## Claims

1. An absorbent article comprising:
a. a first outermost layer for contact with a wearer' s skin or a caregiver' s skin, the first outermost layer comprising a first nonwoven comprising plant-based fibers comprising harvested fibers other than wood pulp, wherein the harvested fibers optionally comprise a post-harvest treatment; and
b. an opposing second outermost layer for contact with the wearer's skin or the caregiver's skin, the second outermost layer comprising a second nonwoven comprising plant-based fibers comprising synthetic fibers;
c. wherein the first nonwoven's bio-based content is from 5% to 100% using ASTM D6866-10, method B; and
d. wherein the second nonwoven's bio-based content is from 5% to 100% using ASTM D6866-10, method B
wherein the bio-based content is the amount of carbon from a renewable resource in the nonwoven as a percent of the mass of the total organic carbon in the nonwoven, as determined by ASTM D6866-10, method B.

2. The absorbent article according to Claim 1, wherein the first nonwoven' s bio-based content is from 10% to 50%, using ASTM D6866-10, method B.

3. The absorbent article according to Claim 1 or 2, wherein the second nonwoven's bio-based content is from 10% to 50%, using ASTM D6866-10, method B.

4. The absorbent article according to any one of the preceding claims, wherein the harvested fibers comprise cotton fibers.

5. The absorbent article according to Claim 4, wherein the cotton fibers are bleached but are devoid of chlorine.

6. The absorbent article according to any one of the preceding claims, comprising a mineral-based odor management material.

7. The absorbent article according to any one of the preceding claims, wherein the plant-based fibers that are synthetic fibers comprise bio-based polyethylene.

8. The absorbent article according to any one of the preceding claims, wherein both the first nonwoven and the second nonwoven comprise petroleum-based synthetic fibers

9. The absorbent article according to any one of the preceding claims, wherein the absorbent article further comprises an odor management material.

10. The absorbent article according to any one of the preceding claims, wherein the plant-based synthetic fibers comprise bio-based polyolefin fibers.

11. The absorbent article according to any one of the preceding claims, wherein the plant-based harvested fibers comprise cotton fibers.

12. The absorbent article according to any one of the preceding claims, wherein each of the first nonwoven and the second nonwoven comprises petroleum-based fibers.

## Patentansprüche

1. Absorptionsartikel, umfassend:
a. eine erste äußerste Schicht für den Kontakt mit der Haut eines Trägers oder der Haut einer Pflegekraft, die erste äußerste Schicht umfassend ein erstes Vlies, umfassend pflanzenbasierte Fasern, umfassend andere geerntete Fasern als Holzfaserstoff , wobei die geernteten Fasern optional eine Nacherntebehandlung umfassen; und
b. eine gegenüberliegende zweite äußerste Schicht für den Kontakt mit der Haut des Trägers oder der Haut des Pflegers, die zweite äußerste Schicht umfassend ein zweites Vlies, umfassend pflanzenbasierte Fasern, umfassend synthetische Fasern;
c. wobei der biobasierte Gehalt des ersten Vlieses von 5 % bis 100 %, unter Verwendung von ASTM D6866-10, Verfahren B, beträgt; und
d. wobei der biobasierte Anteil des zweiten Vlieses von 5 % bis 100 %, unter Verwendung von ASTM D6866-10, Verfahren B, beträgt.
wobei der biobasierte Gehalt die Menge an Kohlenstoff aus einer erneuerbaren Ressource in den Vlies in Massenprozent des gesamten organischen Kohlenstoffs in dem Vlies, wie bestimmt durch ASTM D6866-10, Verfahren B, ist.

2. Absorptionsartikel nach Anspruch 1, wobei der biobasierte Gehalt des ersten Vlieses von 10 % bis 50 % unter Verwendung von ASTM D6866-10, Verfahren B, beträgt.

3. Absorptionsartikel nach Anspruch 1 oder 2, wobei der biobasierte Gehalt des zweiten Vlieses von 10 % bis 50 % unter Verwendung ASTM D6866-10, Verfahren B, beträgt.

4. Absorptionsartikel nach einem der vorstehenden Ansprüche, wobei die geernteten Fasern Baumwollfasern umfassen.

5. Absorptionsartikel nach Anspruch 4, wobei die Baumwollfasern gebleicht, jedoch chlorfrei sind.

6. Absorptionsartikel nach einem der vorstehenden Ansprüche, umfassend ein mineralbasiertes Geruchsmanagementmaterial.

7. Absorptionsartikel nach einem der vorstehenden Ansprüche, wobei die pflanzenbasierten Fasern, die synthetische Fasern sind, biobasiertes Polyethylen umfassen.

8. Absorptionsartikel nach einem der vorstehenden Ansprüche, wobei sowohl das erste Vlies als auch das zweite Vlies erdölbasierte synthetische Fasern umfassen.

9. Absorptionsartikel nach einem der vorstehenden Ansprüche, wobei der Absorptionsartikel ferner ein Geruchsmanagementmaterial umfasst.

10. Absorptionsartikel nach einem der vorstehenden Ansprüche, wobei die pflanzenbasierten synthetischen Fasern biobasierte Polyolefinfasern umfassen.

11. Absorptionsartikel nach einem der vorstehenden Ansprüche, wobei die pflanzenbasierten geernteten Fasern Bauwollfasern umfassen.

12. Absorptionsartikel nach einem der vorstehenden Ansprüche, wobei jedes des ersten Vlieses und des zweiten Vlieses erdölbasierte Fasern umfassen.

## Revendications

1. Article absorbant comprenant :
a. une première couche ultrapériphérique destinée à entrer en contact avec la peau d'un porteur ou avec la peau d'un soignant, la première couche ultrapériphérique comprenant un premier non-tissé comprenant des fibres d'origine végétale comprenant des fibres récoltées autres que de la pâte de bois, dans lequel les fibres récoltées peuvent éventuellement subir un traitement post-récolte ; et
b. une seconde couche ultrapériphérique opposée destinée à entrer en contact avec la peau du porteur ou avec la peau du soignant, la seconde couche ultrapériphérique comprenant un second non-tissé comprenant des fibres d'origine végétale comprenant des fibres synthétiques ;
c. dans lequel la teneur biosourcée du premier non-tissé est de 5 % à 100 % selon le procédé B de la norme ASTM D6866-10 ; et
d. dans lequel la teneur biosourcée du second non-tissé est de 5 % à 100 % selon le procédé B de la norme ASTM D6866-10
dans lequel la teneur biosourcée est la quantité de carbone provenant d'une ressource renouvelable dans le non-tissé en tant que pourcentage de la masse du carbone organique total dans le non-tissé, telle que déterminée selon le procédé B de la norme ASTM D6866-10.

2. Article absorbant selon la revendication 1, dans lequel la teneur biosourcée du premier non-tissé est de 10 % à 50 % selon le procédé B de la norme ASTM D6866-10.

3. Article absorbant selon la revendication 1 ou 2, dans lequel la teneur biosourcée du second non-tissé est de 10 % à 50 % selon le procédé B de la norme ASTM D6866-10.

4. Article absorbant selon l'une quelconque des revendications précédentes, dans lequel les fibres récoltées comprennent des fibres de coton.

5. Article absorbant selon la revendication 4, dans lequel les fibres de coton sont blanchies mais sont dénuées de chlore.

6. Article absorbant selon l'une quelconque des revendications précédentes, comprenant un matériau de gestion des odeurs à base minérale.

7. Article absorbant selon l'une quelconque des revendications précédentes, dans lequel les fibres d'origine végétale qui sont des fibres synthétiques comprennent du polyéthylène biosourcé.

8. Article absorbant selon l'une quelconque des revendications précédentes, dans lequel à la fois le premier non-tissé et le second non-tissé comprennent des fibres synthétiques à base de pétrole.

9. Article absorbant selon l'une quelconque des revendications précédentes, dans lequel l'article absorbant comprend en outre un matériau de gestion des odeurs.

10. Article absorbant selon l'une quelconque des revendications précédentes, dans lequel les fibres synthétiques d'origine végétale comprennent des fibres polyoléfines biosourcées.

11. Article absorbant selon l'une quelconque des revendications précédentes, dans lequel les fibres récoltées d'origine végétale comprennent des fibres de coton.

12. Article absorbant selon l'une quelconque des revendications précédentes, dans lequel chacun parmi le premier non-tissé et le second non-tissé comprend des fibres à base de pétrole.
